(19) Europäisches Patentamt
European Patent Office
Office européen des brevets

(11) **EP 4 548 770 A1**

(12) **EUROPEAN PATENT APPLICATION**

(43) Date of publication:
**07.05.2025   Bulletin 2025/19**

(21) Application number: **23207769.3**

(22) Date of filing: **03.11.2023**

(51) International Patent Classification (IPC):
*A23G 1/42* (2006.01)  *A23L 33/135* (2016.01)
*A23P 10/35* (2016.01)  *A23P 20/10* (2016.01)
*A61K 9/50* (2006.01)  *A61K 35/744* (2015.01)
*A61K 35/747* (2015.01)  *C12N 1/04* (2006.01)
*C12N 11/02* (2006.01)

(52) Cooperative Patent Classification (CPC):
**A23P 10/35; A23G 1/423; A23L 33/135;
A23P 10/30; A23P 20/11; A61K 35/744;
A61K 35/747; C12N 1/04; C12N 1/20; C12N 11/02;**
C12R 2001/225

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Olimp Laboratories spolka z
ograniczona
Odpowiedzialnoscia
39-200 Debica (PL)**

(72) Inventors:
• **KUBAS, Kamil**
  Debica (PL)
• **WIDLAK-KARGUL, Justyna**
  Nagawczyna (PL)
• **MYTYCH, Jennifer**
  Rzeszow (PL)
• **MICULA, Anna**
  Zawada (PL)
• **WIELGOS, Barbara**
  Debica (PL)

(74) Representative: **AOMB Polska Sp. z.o.o.
ul. Rondo Ignacego Daszynskiego 1
00-843 Warsaw (PL)**

(54) **PARTICLE CONTAINING LACTIC ACID BACTERIA, METHOD FOR PRODUCING AND USE THEREOF**

(57)     The invention concerns particles containing lactic acid bacteria, covered with a single, three-component hydrophobic layer of plant origin, in which the hydrophobic layer consists of a mixture of cocoa butter NCB-HDO3-473 and two saturated Fatty acids, in which the content of cocoa Fat in the hydrophobic layer is in the range of 25-29% by weight. The invention also relates to a method For producing particles containing lacticacid bacteria, with a single particle size in the range of 0.18 mm - 1.0 mm, and the use of the particles.

Fig. 1

EP 4 548 770 A1

## Description

### Technical field

The invention concerns particles containing Freeze-dried lactic acid bacteria covered with a single-layer hydrophobic layer. The invention also relates to a quick, repeatable, high-efficiency, and easily transferable method For producing a composition in the Form of protected particles, which contain lactic acid bacteria that are beneficial to the human body and are generally considered to be highly sensitive to moisture, oxidizing agents, elevated temperature or acidic environment and were protected with a specific non-hydrophilic cover to increase their stability during processing and storage of the Finished product.

### Background Art

Including lyophilized probiotic bacteria in Food products is associated with their beneficial effects on the human body. To produce such an effect, however, it is necessary that a sufficiently high amount of them, in a live Form and capable of colonization through consumed Food, reaches the target section of the human digestive system, which is hindered by the aggressive, acidic environment of the stomach. However, before this happens, bacteria passing through various stages of Food product production, transport, and Final storage are exposed to Factors that reduce their beneficial activity, such as temperature, humidity, or oxygen, which Further reduces the chances of ensuring adequate survival. The chemical composition of the Food products in which they are Found is also important in terms of ensuring adequate survival of bacteria, which only highlights the difficulty of this task and limits the number of Food product categories in which it is possible to maintain a sufficiently high number of bacteria throughout the shelf life.

The use of high-barrier packaging only partially solves the problem of too short viability of bacteria in Food products because it provides protection only For storage conditions while being an expensive solution. However, it does not protect the sensitive bacterial material against the direct influence of environmental conditions at various stages of production before packaging, and it does not protect against the harmful effects of the acidity of the human stomach. Hence, there was a need to develop a solution ensuring the creation of a protective layer directly on the surface of bacteria in the Form of lyophilizate to give or increase their resistance to all the Factors mentioned above. Therefore, many scientists have started research in this Field.

Various solutions have been proposed, the aim of which was to protect bacteria in the Form of lyophilizate with a protective layer or layers.

Patent EP 3205216 B1 discloses the protection of sensitive probiotic material with more than one protective layer, where the core and the First protective layer are a polymer of sodium alginate or pectin dissolved in water in an appropriate concentration, and the next, inner layer is a polymer of sodium alginate or pectin with the addition of another probiotic strain dissolved in water in an appropriate concentration.. The solutions prepared in this way are then injected using a special two-nozzle system (internal nozzle + ring nozzle) into a solution containing calcium chloride at a concentration of 20g/L. The polymer of sodium alginate or pectin reacts with calcium chloride to create small balls with a soft structure containing probiotic bacteria, which are Further dried and then covered with a hydrophobic layer. The disadvantage of this solution is the addition of bacteria to aqueous solutions containing dissolved ingredients intended to perform a protective Function, which must result in an increase in the water activity in the probiotic material as a result of rehydration and thus must result in a reduction in the number of live probiotic bacteria, even though the next stage involves drying the obtained granules in to remove excess water. The use of water-soluble ingredients as a protective layer directly in contact with the probiotic material is unjustified due to the sensitivity of such material; adding subsequent layers, even hydrophobic ones, cannot stop the processes that started as a result of the hydration of lyophilized bacteria. This patent also does not present long-term stability results during the storage of the obtained microcapsules.

In turn, patent US 110396372 B2 talks about a multi-layer protective composition consisting of: 1) a core containing probiotic bacteria (*Bifidobacteria* - BB12), trehalose dihydrate, maltodextrin DE15 and L-cysteine HCl monohydrate, which is obtained by homogenizing the mixture of these ingredients by mixing in the Innojet Ventilus machine and then combined into larger agglomerates by spraying molten lauric acid, 2) the innermost coating hydrophobic layer consisting of molten lauric acid, 3) the next two sealing layers containing water-soluble sodium alginate, the two-step application of this layer results From the need to obtain an appropriate increase in this layer, 4) the next two layers applied in two stages containing Na-carboxymethylcellulose and polyethylene glycol (PEG 400) to obtain an appropriate increase. The disadvantage of this solution is the multitude of stages resulting in long exposure of bacteria to stressful conditions, and above all, exposing them to several increases and decreases in temperature during the application of subsequent layers, which require the evaporation of water From the surface of the produced granule. Although the results of bacterial cultures after subsequent stages of preparation of the composition discussed in the patent did not indicate a significant reduction in their viability as a result of the protection process, no data were provided on the viability of bacteria during Further storage of such material, and above all, after passing the material thus protected through an aggressive environment of the human stomach. Therefore, it cannot be clearly stated that such protection of the material fulfills its Function in the context of delivering lyophilized probiotic cells in a live Form to the target site of colonization in the human digestive system throughout its shelf life or the product containing it. Damage to bacterial cells that may occur as a result of multi-stage processing may be reflected in the reduced ability to Form colonies at Further stages of storage, making the material less durable. All the more so because the proposed solution involves the use of aqueous solutions which, despite being sprayed onto a material previously protected with a hydrophobic layer, may lead to the gradual awakening of probiotic bacteria From the state of anabiosis due to the penetration of water through microcracks in the hydrophobic barriers composed only of saturated Fatty acids, which after hardening they are usually Fragile. The complexity of the process also makes it less advantageous in terms of the costs of energy or human resources needed to produce a single batch of semi-finished products in the Form of protected probiotic material.

The document WO 2002058735 A1 describes a method of protecting probiotic bacteria by spraying only aqueous solutions of enteric coatings based on polymers and other ingredients such as sugars or proteins. Unfortunately, the characteristics of the properties resulting From the composition of such coatings do not allow the creation of a barrier on the surface of the coated probiotic material that protects against moisture and oxygen but gives it the character of increased resistance to the acidic environment of the human stomach. Therefore, probiotic material with such a composition usually requires the application of additional layers, which again refers to the issue of the unfavorable impact of production due to the complexity of the process on the ability of probiotic bacteria to colonize in the human digestive system throughout its shelf life or the product containing it.

Document US 6900173 B2 describes an attempt to protect probiotic bacteria by application to a multivitamin protein bar, specifically to a mixture of sugar syrup and several other ingredients. The Final product was created by extruding and cutting the mass-produced. However, no results were presented indicating the survival of bacteria in the manufacturing process or extended stability as a result of long-term storage tests.

Document US 4888171 A describes a product in the Form of a sugar core directly covered with a mixture made of Freeze-dried bacteria and other binder ingredients with a melting point in the range of 25-60°C. The Final product was obtained by evaporating the binder together with the bacteria medium in a granulation chamber with a temperature ranging From 25 to 55°C. Since ensuring a sufficiently high survival rate For probiotic bacteria is extremely difficult at temperatures equal to or even lower than human body temperature, it seems inappropriate to use temperatures higher than 36.6°C For the process of protecting bacteria. The use of higher temperatures of the Fluidizing gas and the process bed For probiotic bacteria results in a partial reduction of the total number of live bacteria in the material or their damage, which consequently translates into an accelerated decline in viability during storage, thus shortening the durability of the obtained product.

Patent EP 1265983 B1 discloses a method of coating dehydrated, living microorganisms by applying a homogeneous hydrophobic layer containing a substance selected From Fatty acids, waxes, or mixtures thereof, with particular emphasis on stearic and palmitic acid. The use of a protective coating consisting of saturated Fatty acids or other hydrophobic ingredients with a high content and a high melting point, increases the susceptibility of such a layer to the Formation of microcracks after hardening. Defects in the coating surface may allow water to penetrate inside, which leads to the gradual awakening of probiotic bacteria From the state of anabiosis and then their death. This draws attention to the need to use an appropriately adjusted amount of ingredients with a lower melting point in hydrophobic coatings, which results From the content of small amounts of unsaturated Fatty acids in such ingredients, which does not reduce the resistance of the hydrophobic coating to oxidizing agents. The document does not disclose results that would indicate a reduction in the hygroscopic nature of the protected probiotic bacteria or maintaining low water activity, so it cannot be concluded that the applied coating protected against the adverse effects of ambient humidity.

The protection process described in document EP 1265983 B1 is characterized by the Fact that said hydrophobic substance is injected at a temperature higher than its melting point, said injection temperature being between 30 and 120°C, and an air stream at a temperature between 10 and 50°C, so that the temperature in the chamber does not exceed by more than 5°C the viability temperature of said microorganisms. The coating process uses standard equipment called a granulator and the microorganisms to be coated are living cells in the Form of a lyophilized product. The proportion of the coating material in relation to the number of microorganisms is From 10 to 99% by weight, preferably From 30 to 80%. Since ensuring a sufficiently high survival rate For probiotic bacteria is extremely difficult at temperatures equal to or even lower than human body temperature, it seems inappropriate to use gases at temperatures higher than 36.6°C For the process of protecting bacteria. It also seems unreasonable to allow the bed to be maintained at a temperature of up to 5°C above the optimal temperature For bacterial growth. Such a selection of process parameters, even if it does not lead to a decrease in viability during and immediately after it, results in partial damage to bacterial cells, which during Further storage results in an accelerated decrease in the total number of live bacteria in the material, thus shortening its shelf life. It is worth noting that the patent description does not contain results From long-term stability tests that would confirm that the proposed solution extends the durability of the probiotic material. Therefore, it cannot be clearly stated whether the proposed solution is effective.

The document EP 1265983 B1 presents results indicating the achievement of Features of increased resistance to physicochemical stresses, such as heat, acidity, or compression, but does not present results indicating increased resistance to temperature and humidity during long-term stability tests, hence the effectiveness of the proposed solution cannot be confirmed in the entire shelf life of the material or product containing it. The use of these particles in the Food industry was also presented, by adding them to products such as cereals, confectionery, or powdered milk, although only the results were presented concerning tests of the application of protected bacteria to powdered milk, hence the effectiveness of the developed solution in the application tests e.g. For confectionery products cannot be clearly stated

There is no effective solution known in the state of the art that would ensure that probiotic bacteria, after processing and dehydration, retain all the properties necessary to work effectively in a Food product, without losing their viability as a result of the production process. Additionally, no process has been disclosed that provides a Fast, repeatable, high-efficiency method that can be easily transferred to a production scale For producing particles.

The solution in question aimed to overcome the above-mentioned problems related to the effective use of probiotic bacteria, among others, in a Food product, as well as to provide a method For producing such coated particles.

Surprisingly, it turned out that the solution according to the independent claims of this application makes it possible to achieve this goal.

The subject of the invention is a particle containing Freeze-dried lactic acid bacteria, covered with a single, three-component hydrophobic layer of plant origin, in which the hydrophobic layer consists of a mixture oF:

    a. cocoa butter NCB-HDO3-473,

    b. two saturated Fatty acids,

wherein the cocoa Fat content in the hydrophobic layer is in the range of 25-29% by weight.

Preferably, the lactic acid bacteria belong to the *Lactobacillus rhamnosus* GG strain (ATC53103) and the total amount of Freeze-dried bacteria in the particle is 45-55% by weight, and the hydrophobic layer is 45-55% by weight of the particle.

Preferably, the melting point of the hydrophobic layer is in the range From 34°C to 64°C.

Preferably, one of the saturated Fatty acids has a melting point in the range of 57-64 °C, the other saturated Fatty acid has a melting point in the range of 53-60 °C and the NCB-HDO3-473 cocoa butter has a melting point in the range of 34-36 °C.

Preferably, the content of cocoa Fat, a Fatty acid with a lower melting point, and a Fatty acid with a higher melting point were selected according to the weight proportion, respectively: 1.125: 1.0: 2.0416.

Preferably, the saturated Fatty acids are selected From the group consisting of palmitic acid and stearic acid.

The invention also relates to a method For producing particles containing lactic acid bacteria, with a single particle size in the range of 0.125 mm - 1.0 mm, which includes the Following steps:

    a) preparation of a hydrophobic coating mixture consisting of cocoa butter and two saturated Fatty acids,

    b) conditioning of lactic acid bacteria by heating to a temperature of 20-22 ° C For at least 2 hours,

    c) carrying out preliminary process,

    d) cooling,

    e) carrying out proper process,

wherein the total efficiency of the process is at least 90% and the water content in the obtained particles is not more than 0.7%.

Preferably, step a) includes melting all the ingredients, with the melting being carried out in the order resulting From the melting temperatures of the individual ingredients From the lowest to the highest, at a temperature of 110°C, Followed by sterilization of the molten mixture at a temperature of 121°C.

Preferably, the content of individual components of the hydrophobic layer is in the weight proportion: 1.125: 1: 2.042, respectively: cocoa butter, a Fatty acid with a lower melting point, and Fatty acid with a higher melting point.

Preferably, the unsaturated Fatty acids are selected From the group consisting of palmitic acid and stearic acid.

Preferably, step c) is carried out by hot melting in a Wurster coating system providing spray From below or in a Fluidized chamber providing spray From above, and consists of two stages:

    i) the duration of the First stage is 5 minutes, the spraying dose of the mixture From point a) is 15 g/min, the inert gas Flow is 20-25 $m^3$/h, the atomizing pressure is 1 bar, the inlet inert gas humidity is in the range of 0, 0-0.1 g/kg,

    ii) the duration of the second stage is From 7 to 18 minutes, the spraying dose of the mixture From point a) is 20 g/min. inert gas Flow: 25-45 $m^3$/h, atomizing pressure is 1 bar, inlet inert gas humidity is in the range of 0.0-0.1g/kg

Preferably, cooling in step d) is carried out in an inert gas Flow of 35-45 $m^3$/h For 10 minutes, the humidity of the inlet inert gas is in the range of 0.0-0.1g/kg.

Preferably, process in step e) takes place within 16 to 23 minutes, the spraying dose of the mixture From point a) is 15-20 g/min, the inert gas Flow is 35-45 m³/h, the atomizing pressure is 1 bar, the inert gas humidity inlet is in the range of 0.0-0.1g/kg.

Preferably, the percentage distribution of Fractions with different particle sizes is as Follows: the Fraction with the size of a single protected particle in the range of 0.16 mm - 0.6 mm - constitutes 85% by weight of the total material, the Fraction with a particle size in the range of 0.125 mm - 0.16 mm - constitutes less than 4% of the total, and the Fraction with a particle size in the range of 0.6 mm - 0.8 mm - constitutes 10-11% by weight of the total material.

In another aspect, the invention concerns the use of particles For use in product Forms such as chocolate, chocolate products, bars, products in liquid and semi-liquid Form, capsules, and sachets with ZIP or stick closures.

In the First aspect, the present invention concerns a protected particle containing lactic acid bacteria in a lyophilized Form, which is beneficial to the human body. The present invention is characterized by increased resistance to elevated temperature, humidity, and the acidic environment of the human stomach, demonstrated in long-term stability tests, considering parameters such as the ability to Form colonies and viability. An artificial digestive system was used to determine the resistance to the acidic environment of the stomach, which more faithfully reflects the conditions in the human body, giving the results a greater level of credibility. Measurements of changes in water content and water activity throughout long-term storage tests and determination of the level of hygroscopicity compared to unprotected material were also used as auxiliary parameters proving that the particles thus protected have increased resistance to unfavorable conditions of the storage or production environment. The combination of all these Features seems to be able to ensure the delivery of appropriately high doses of biologically active probiotic bacteria to the intestine in products that are dietary supplements, fortified Foods, Food For special medical purposes, Food For people with specific nutritional needs in the prevention of lifestyle diseases, including overweight and obesity as well as in inflammatory bowel diseases or the diets of athletes. The discussed solution does not require the use of more than 55% by weight of the substrate in the entire composition, which makes the process cheaper, shorter, and gentler For probiotic bacteria.

In the second aspect, the present invention concerns a Fast, repeatable, high-efficiency, and easily transferable method For producing a composition in the Form of protected particles, which contains lyophilized bacteria beneficial to the human body and which are generally considered to be characterized by high sensitivity to moisture, oxidizing agents, elevated temperature or acidic environment and are protected with a specific non-hydrophilic cover to increase their stability during processing and storage of the Finished product.

The composition in question can also be called a semi-finished product because this term describes a product that has passed all stages of initial production and is one of the input materials For the production of Final Finished products.

This solution enables the delivery of appropriately high doses of probiotic bacteria to the intestine. Thus, it refers to the use in products that are dietary supplements, fortified Foods, Food For special medical purposes, Food For people with specific nutritional needs in the prevention of lifestyle diseases, including overweight and obesity, as well as in non-specific inflammatory bowel diseases or the diets of athletes.

The present invention provides a composition of lactic acid bacteria which, due to the presence of a complex non-hydrophilic layer with a relatively high transition temperature From the solid to the liquid state and vice versa, is characterized by resistance to increased temperature as well as to its Fluctuations that may occur during the daily cycle, during storage and use the discussed composition in the production processes of Food products. The composition of the applied non-hydrophilic layer significantly limits the impact of unfavorable oxidizing Factors on the intermediate product due to the high content of saturated Fatty acids (not less than 90.15% by weight). The composition is also characterized by lower susceptibility to absorb water vapor From the air compared to lyophilizate of lactic acid bacteria and is characterized by lower hygroscopicity.

The present invention provides a composition that contains: 1) lyophilized bacterial material, where the total amount of bacteria may constitute 1-99% (w/w) of the entire composition 2) a non-hydrophilic, three-component, dual-functional granulating and protective cover For bacteria, where each of the components of this cover characterized by a transition temperature From liquid to solid and vice versa lower than 64°C and higher than 34°C, containing at least two saturated Fatty acids and cocoa butter NCB-HDO3-473.

The dual-function granulating and protective casing proposed in the invention contains NCB-HDO3-473 cocoa Fat, which, due to its low melting/solidification temperature and low content of unsaturated Fatty acids, provides greater plasticity and Flexibility in the sense of lower susceptibility to the Formation of microcracks in the casing or its crumbling than in casings consisting only of saturated Fatty acids with higher melting points. This directly results in better barrier properties than in covers consisting only of saturated Fatty acids. The appropriately selected percentage of NCB-HDO3-473 cocoa Fat in the granulating and protective casing ensures that it has the described properties that improve the durability of the bacterial ingredients. Moreover, a casing with such a selected cocoa Fat content (preferably in the range of 25-29% of the entire casing) combined with an appropriate setting of the temperatures of the process gases (atomizing gas, Fluidized gas) and the bed temperature at each stage of its atomization allows it to be simultaneously granulating and covering properties.

The non-hydrophilic granulating and protective layer contains at least two saturated Fatty acids, one of which is an acid with a melting point lower than 64°C and preferably higher than 57°C, and the other is an acid with a melting point lower than 60°C and preferably higher than 53°C. Enriching the above system with NCB-HDO3-473 cocoa butter, with a melting point lower than 40°C and higher than 30°C, and more preferably lower than 36°C and higher than 34°C, makes the coating layer complete. It is characterized by a transition temperature From solid to liquid and vice versa in the range of 48-52°C. The amounts of individual components of the bifunctional granulating and protective coating were selected according to the weight ratio of 1.125:1.0:2.0416, where the largest amount is the Fatty acid with the highest melting point, and the smallest amount is the second Fatty acid with a lower melting point.

Such a selected composition and proportion of the protective non-hydrophilic layer ensures resistance to high temperatures thanks to the presence of palmitic and stearic acid, while at the same time making it resistant to crumbling after solidification thanks to the content of cocoa Fat NCB-HDO3-473.

The invention in question ensures obtaining protected particles containing Freeze-dried probiotic bacteria with increased resistance to elevated temperature, oxidizing agents, and air moisture, which should preferably be produced in the hot melting process in a Wurster coating system with bottom spray; in an alternative variant, it can also be produced in a classic Fluidizing attachment with spraying From the top using the same process parameters (except For the parameter of the gap width between the guide sleeve and the screen, this parameter does not occur when working with a classic Fluidizing attachment). The use of the Wurster coating system allows For stable circulation of probiotic material. The Flow of gas through a specially designed column with an internal guide sleeve, when properly positioned above the surface of the lower sieve, causes the particles of lyophilized bacteria to be evenly sprayed with a dual-function granulating and protective cover.

The manufactured semi-finished product has the Form of loose, Fine particles, with a single particle size of not more than 1.0 mm and not less than 0.125 mm, with the vast majority of the particles having a Fraction with a particle size ranging between 0.16 mm and 0.6 mm-this Fraction constitutes 85% by weight of the total material. In turn, the Fraction with a particle size in the range of 0.125-0.16 mm constitutes a negligible percentage of the total, i.e. less than 4%, and the Fraction with a particle size in the range of 0.6-0.8 mm constitutes 10-11% by weight of the total material. The obtained particle Fraction is a material with a very even size of a single particle, as 95% by weight of all particles is in the range of 0.16-0.8 mm. The size of a single protected particle is determined based on the results of sieve analysis. This effect was achieved with the content of non-hydrophilic shield components at the level of 45-55% by weight of the entire composition. This particle size distribution, as well as the resulting sufficiently good powder Flow coefficient, i.e. Hausner Ratio, combined with an appropriately high bulk density, makes the semi-finished product suitable For use in automated dosing systems without the need to use significant amounts of other ingredients (fillers) that improve these physical properties to obtain equal doses of the protected intermediate with probiotic bacteria per unit of Food products From the previously mentioned categories.

The developed composition, thanks to all the properties described in the previous points, is suitable For use in products in the Form of a powder mixture packed in a capsule, sachet, plastic jar, bag with a ZIP closure, or in a stick sachet, which can be made of multilayer thermoplastic polymer alloys, aluminum or its admixture or with an admixture of a paper layer, and is also suitable For use as an additive to chocolate to produce another semi-finished product suitable for application in Food products with high watercontentand high-water activity For lyophilized probiotic bacteria. Providing bacteria with thermal resistance thanks to the applied composition will also enable their safe application of bars, chocolate, or other chocolate products.

The presence of only one non-hydrophilic layer in the composition, which simultaneously performs two Functions, granulating and coating, does not cause excessive dilution of the raw material, which ensures the supply of lactic acid bacteria that cause a documented health-promoting effect when using small doses of the protected intermediate [mg].

The method of producing the discussed semi-finished product in the Form of single-layer microcapsules ensures no loss of viability in the probiotic material compared to the viability recorded For the input raw material. This is confirmed by the collected results of cytometric analysis performed For several manufactured batches. Moreover, the production method does not increase the water activity and moisture content of the material, which Further confirms the lack of negative impact of the process on the biological material and its activity.

The non-invasive nature of the process of protecting lactic acid bacteria with a hydrophobic layer was achieved, among others, thanks to the use of an atmosphere of dried inert gas ($N_2$) with a moisture content of 0.0-0.1g/kg. This process environment was achieved by incorporating a nitrogen generator into the line, which also has a drying Function.

Obtaining mild production conditions in the discussed invention was also achieved thanks to the appropriate selection of process parameters such as the temperature of the inlet and atomizing gas at the outlet From the atomizing nozzle, the velocity of the inlet gas, and the dose of the sprayed granulating and protective cover per minute. The use of optimized settings results in a low bed temperature during the process of applying the non-hydrophilic layer, which prevents the lyophilizate From drying out and, consequently, accelerating the processes of moisture absorption From the environment, and also prevents protein denaturation processes that may occur as a result of too high temperatures.

In the preferred method of implementing the invention, it is also crucial to adjust the temperature and humidity conditions in the room of the workshop For the production of the semi-finished product in the Form of protected particles so that the hygroscopic raw material, i.e. the lyophilizate, does not absorb moisture From the environment at the weighing stage, which could later lead to the awakening of the cells of the lyophilizate of lactic acid bacteria. In the preferred method of implementing the invention, all operations with the lyophilized product should be carried out at a temperature below 22°C and a humidity below 20%. Equipping the room with HEPA Filters and laminar airflow additionally protects against cross-contamination.

The analysis of the input material (lyophilizate) and the produced semi-finished product (protected particles) extended by a viability test performed on a Flow cytometer showed that bacteria From the protected particles had a more beneficial effect on the human intestinal microflora by supplying a larger total number of live lactic acid bacteria to the intestine, which was able to create colonies compared to commercial microencapsulated lyophilizate of lactic acid bacteria.

The discussed invention in the Form of protected particles after passing through the intestinal passage of the SHIME® artificial digestive system showed greater viability and the ability to Form colonies than the commercial microencapsulated lyophilizate of lactic acid bacteria. The commercial microencapsulated lyophilizate after passing through the artificial digestive system showed a viability of 2.62% with a colony-forming ability of $9.33 \times 10^7$ CFU/g. In turn, the discussed invention in the Form of protected particles, subjected to an identical experiment, showed a viability of 18.73% with a colony-forming ability of 3.44x$10^8$ CFU/g. These results were recorded in tests carried out For samples stored in a climatic chamber at 25 degrees C/60% humidity. A similar trend was noted For samples stored in variable conditions, where temperatures ranged From 20-24 degrees Celsius and humidity ranged From 15-50%.

For a commercial microencapsulated lyophilizate, the viability after passing through the intestinal passage was 7.35% of the total number of bacteria with a colony-forming ability of $7.8 \times 10^7$ CFU/g, while For the discussed invention with a viability of 22.38%, the colony-forming ability was at the level of $1.37 \times 10^8$ CFU/g.

The particles according to the invention contain stabilized lactic acid bacteria *Lactobacillus rhamnosus* (ATCC 53103). The coated particle contains one hydrophobic layer, whose task is to prevent water From penetrating inside the microcapsule, which is the main Factor in awakening bacteria From the state of anabiosis. *Lactobacillus rhamnosus* bacteria are initially granulated and surrounded by applying part of the granulating and protective cover provided For in the invention. After the initial hardening of the said particles as a result of the cooling stage, they are then covered with the remaining amount of the granulating and protective casing, which consists of palmitic acid, stearic acid, and cocoa Fat NCB-HDO3-473.

The main purpose of protection is to provide bacteria with protection against moisture (water vapor) and oxidizing Factors. Such protection allows For the longest possible stability during storage of particles as an intermediate product and is intended to increase the resistance of probiotic bacteria when applied to products (additionally without the use of refrigeration conditions) that are dietary supplements, fortified Foods, Food For special medical purposes, Food For people with specific nutritional needs. in the prevention of lifestyle diseases, including overweight and obesity, as well as in the case of non-specific inflammatory bowel diseases or products dedicated to athletes' diets. The described protection is also intended to protect against the destructive effects of digestive juices during their passage through the human digestive system, thereby increasing the number of bacterial components that can reach the small and large intestines in an active Form.

The semi-finished product in the Form of protected particles containing probiotic bacteria, due to the appropriate method of applying a cover with the addition of cocoa Fat and the appropriate percentage of its content in the semi-finished product, not only shows increased resistance to changing temperature conditions during storage but also shows resistance to the increased temperature that may occur during storage. Further stages of processing of the semi-finished product in question (e.g. when enriching its composition with dark chocolate ingredients, where the Final semi-finished product in this particular example is in the Form of a decor or chocolate sprinkle of any shape but relatively small sizes (up to several mm in diameter), in which the dark chocolate ingredients can be used as additional protection against bacteria or as a carrier of an attractive taste).

The subject matter of the invention, in embodiments, is reproduced in a drawing in which:

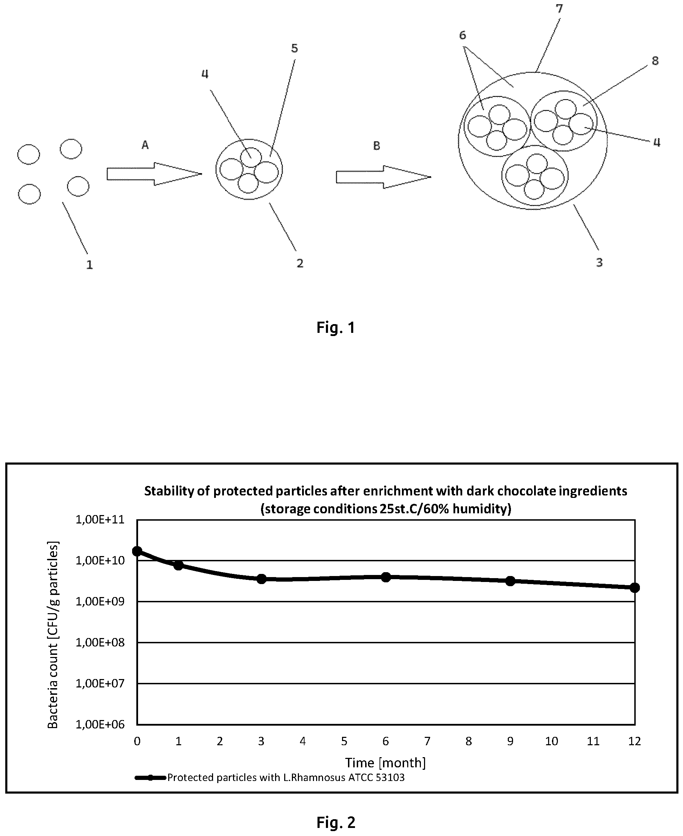

Fig. 1 shows a diagram of the Formation of a protected particle containing lactic acid bacteria, in which the individual references mean: A - initial process, B - proper protection, 1 - single or loose particles of the unprotected lyophilizate, 2 - particles containing the partially protected lyophilizate and combined into larger agglomerates, 3 - protected particle containing interconnected particles of the initial particles surrounded by an appropriate protective layer, 4-lyophilized particle, 5 - pre-hardened combined granulating and protective casing, 6 - dual-Functional granulating and protective casing, 7 - particle of the protected particle: semi-Finished product after initial granulation and proper granulation with a non-hydrophilic cover, 8 - a molecule of particle.

Fig. 2 shows the stability chart of the protected particles after enriching with dark chocolate ingredients (storage conditions (25 °C, 60% humidity).

Fig. 3 shows the storage stability graph at 25 °C, 60% humidity.

Fig. 4 is a graph illustrating the rate of change of water content during storage at 25 °C, 60% humidity.

Fig. 5 is a graph illustrating the rate of change of water activity during storage at 25 °C, 60% humidity.

**Embodiments of the invention:**

**Example 1. Method of producing particles**

**Preparation of the laboratory**

It is necessary to obtain appropriate temperature and air humidity conditions in the laboratory where the process of preparing samples and securing them will take place. The minimum that must be met is the temperature below 25 ° C and the humidity below 35%. However, in a more Favorable variant, the air temperature should be maintained in the range of 20-22 ° C and the air humidity should not exceed 20%. Providing appropriate conditions protects the input material, which is the hygroscopic lyophilizate of *Lactobacillus rhamnosus* bacteria (ATCC 53103), From absorbing moisture From the air at the stage of preparing raw material samples For the process of protection with a bifunctional granulating and protective layer. Optimal conditions in the preferred embodiment are achieved by reducing air Flows through the room in combination with installing air conditioners and air dehumidifiers in the ventilation system. Do not attempt any operations with open probiotic material if the room conditions do not meet the requirements.

Before each campaign or individual production of the invention in question, the room in which the process is carried out must be cleaned to prevent cross-contamination of the intermediate. The Following products are cleaned with appropriately selected cleaning and disinfecting agents: Floors, walls, and ceilings, including hard-to-reach places, glass, including door glass, countertops, cabinets, handles, table legs, protruding parts of contacts, doors, room handles and other devices located in the workshop room, taking into account hard-to-reach surfaces, top and side surfaces, the same washing procedures apply to the passenger lock leading to the workshop.

Before each campaign or individual production, all granulator parts, devices, and auxiliary tools that may come into contact with the input material are subject to a washing and disinfection procedure, the effectiveness of which is confirmed by determining the level of microbiological and organic contaminants expressed in the unit [RLU/25cm$^3$] using a luminometer. A low RLU level indicates the cleanliness of the analyzed surface. The critical value indicating excessive contamination of the analyzed surface was set at 50RLU/25cm$^3$ and was established based on validation concerning reference methods.

For each campaign or single production, a set of Filters should be used: two inlets and one outlet, intended only For working with probiotic material.

Before each campaign or single production, ensure the appropriate protective kit For the operator responsible For the production (apron, trousers, shoes, cap, mask, glasses, shoe covers) and its cleanliness to minimize the risk of cross-contamination in the product that may reduce the biological activity of the produced products. microcapsules protected with a non-hydrophilic layer.

**Stage 1** - **preparation of a hydrophobic coating mixture consisting of cocoa butter and two saturated fatty acids:**

Before weighing and melting the components of the protective layer, ensure the sterility of the borosilicate glass bottle and the cap in which the mixture is prepared. The sterility of the vessel is achieved by treatment with a temperature of 121 ° C For 15 minutes under reduced pressure. The prepared vessel For the hydrophobic mixture can be placed in an oil bath (optionally with a magnetic stirrer) to start melting the components of the cover.

The preparation of the hydrophobic mixture, which is also a granulating and protective agent with a melting/solidifying temperature in the range of 48-52 °C, is carried out by melting its components in the appropriate sequence at an oil bath temperature setting in the range of 90-100 °C. It is best to select the amount of melted ingredients included in the protective layer to maximize the use of the 2L capacity of the cylinder. This is important in terms of efficiency in terms of the number of processes carried out during one work shift. When selecting the amount of ingredients, take into account the Fact that their volume increases as the temperature increases. The maximum amount of protective layer ingredients that can Fit in a 2-liter bottle is 1700g. This amount allows two granulation processes to be carried out without the need to add the granulating and protective mixture and at the same time without unnecessary losses.

It is best to melt the components of the protective casing, i.e. cocoa butter, stearic acid type 50, and palmitic acid 98%, in the order resulting From their melting temperatures (from the lowest to the highest). Melting is carried out by starting by melting the cocoa Fat, then the palmitic acid, and Finally the stearic acid, you should not melt everything at once. This is intended to make the stage of preparing the casing as quick and repeatable as possible, without the problems that could occur when melting in the reverse order, where the ingredients with a higher melting pointwould liquefy more slowly, creating dense and aerated masses From the mixer in the initial phase of melting. Mixing is carried out For at least 30 minutes to ensure that the ingredients are well combined. The granulating and protective layer prepared in this way should be sterilized to prevent possible cross-contamination of the lyophilized lactic acid bacteria with the raw materials included in the coating. After sterilizing the system, it should be heated to 110 °C, which should be maintained until the initial granulation stage and actual granulation are completed. The bottle with the system should be opened immediately before loading the *L. rhamnosus* lyophilizate into the granulator column.

**Stage 2** - **Conditioning of the input material:**

At least 2 hours before placing the lyophilizate in the granulator column, a specific amount should be weighed to condition the input material. The most preferred size of the bed at the entrance to the process should be in the range of 650-700g if a Wurster column with a capacity of 6L or a classic Fluidized bed column with a capacity of 6L is used. Conditioning is the slow bringing of the temperature of the material, in this case biological material, to the ambient temperature as a result of gradual transfer From the place of initial storage (-20°C), through a refrigerator with an internal temperature of 4°C, to the room of the encapsulation laboratory (20-22 °C). In the most advantageous variant, the material should be transferred aftersecuring the weighed material with a double polyethylene bag, which, after sucking out the air, is placed in a bag with an aluminum layer and a ZIP closure. Conditioning the material For an hour at a temperature of 4°C and then at 20-22°C ensures that the temperature of the lyophilizate at the start of the initial granulation process is in the range of 20-22°C (measurements of the sensor inside the granulator). Conducting conditioning in this way protects the sensitive lyophilizate against sudden temperature changes, and the influence of oxygen and moisture even before protection, thus preventing a decline in its quality and biological activity.

**Preparation of the granulator and auxiliary equipment for the initial granulation process**

After weighing the necessary amount of lactic acid bacteria and sending it For conditioning, you should turn on and adjust the heaters that are part of the hot melting set. They are responsible For heating the atomizing air to a specific temperature and maintaining the bifunctional granulating and protective layer at a constant temperature during its peristaltic Feeding to the atomizing nozzle. In a specific embodiment of the discussed invention, the heater settings For the pipe supplying the non-hydrophilic mixture should be 110°C and 160°C For the heater responsible For heating the atomizing air and nozzle elements. The use of these specific settings prevents the mixture From solidifying too early when spraying onto the lyophilized lactic acid bacteria, and also prevents it From solidifying in the supply line on the suction side (between the vessel with the non-hydrofolic mixture and the pump) and on the pressure side (between the peristaltic pump and the spray gun covered by a specially adapted insulating cover that prevents heat loss through heated parts) and in the atomizing nozzle. Once the heaters have achieved specific settings, you can turn on the nitrogen generator and then Flow the inert inlet gas through the Wurster column, which will Force it to Flow through the lines supplying the atomizing gas, causing it to start heating to the appropriate temperature. In a specific embodiment of the invention, the temperature of the heated atomizing gas at the outlet From the nozzle should not be lower than 50°C, in a more preferred variant it should be higher than 60°C but lower than 70°C. The atomizing gas heating stage should not be started earlier than 45 minutes before the end of the input material conditioning stage.

**Starting the nitrogen generator**

The generator should be started approximately 3 minutes before starting the inlet gas Flow on the granulator panel to Fill the pipe system with inert gas. Coupling the nitrogen generator with a Fluidized bed granulator, in addition to using the inert Nz gas atmosphere For the process (at a concentration of not less than 98%), also allows it to be carried out in conditions of reduced humidity to the level of 0-0.1g/kg. The water content level in the column is read From the granulator control panel. The stage of heating the critical elements, and especially the atomizing gas, described in the previous section of the discussed invention, should be carried out For at least 30 minutes, and in the most preferred variant, 45 minutes, with an inlet inert gas Flow of 20-25 m$^3$/h. The same value of inlet air Flow should be used at the start of the stage of initial granulation of the lyophilizate.

The use of inert gas in the process is an alternative to air, but only in the case of protecting the bacterial input material, which is not highly sensitive to oxygen. However, in most cases of bacterial materials, they are characterized by high sensitivity to the effects of oxygen (it is toxic to them and causes a rapid decline in biological activity, e.g. in the case of anaerobic bacteria), as well as to the effects of water contained in the air due to their hygroscopic nature. Therefore, the use of nitrogen as a process gas is justified when working with such material so as not to reduce its biological quality before it is covered with a protective cover.

The combination of the generator with the granulator allows, in addition to the use of an inert gas atmosphere, to reduce the water content to 0%. In the case of bacterial materials, increasing the water content or water activity as a result of absorbing moisture From the air starts the processes of cell revival and, consequently, death, hence ensuring such a process atmosphere is crucial to achieving no quality losses in the protected semi-finished product compared to the input material. This is especially true since increasing the water content or water activity in the protected material as a result of the process could hurt shortening its durability. Therefore, in the discussed invention it is possible to Freely switch to operation in an atmosphere of dried inert gas or dry air, and the choice of operating mode depends on the input material with which one is working.

## Stage 3 - **Initial process**

After conditioning the input material For 2 hours and heating the critical elements and the atomizing gas For 45 minutes, a sequence of activities should be initiated to start the initial granulation stage and then granulation with a complex non-hydrophilic mixture. These activities should be performed as quickly as possible to prevent excessive cooling of previously heated elements. To do this, you need to: 1) turn off the Flow of inert gas through the granulator in the opposite order than when starting its Flow, 2) unseal the granulator chamber, 3) gently pour the weighed amount of probiotic bacteria lyophilizate into the Wurster coating system and close it carefully to avoid excessive compaction powder, 4) reseal the granulator chamber, 5) set the initial parameters of the initial granulation process, 5) start the Flow of inert gas through the granulator according to the sequence described in the point above, 6) adjust the operation of the bed (input material) by setting the appropriate width of the gap between the sieve (at the bottom of the Wurster coating system) and the sleeve (above the sieve, which is responsible For the proper Fluidization of particles inside the attachment). In the most Favorable production variant, the gap width at the start of the initial granulation stage should be in the range of 8-12 mm.

After approximately 3 minutes of circulation of the bed inside the granulator column, start spraying the complex bifunctional granulating and the protective mixture heated to 110°C according to the Following parameters (it will also be correct to heat the complex mixture to temperatures in the range of 110-125°C as it does not cause changes in the bed temperature in during the pregranulation stage due to drastic cooling of the mixture at the outlet From the atomizing nozzle) The Following parameters apply For the First 5 minutes of the pregranulation stage to minimize the amount of input material and any solidified particles of the granulating mixture that could reach the product Filter if a larger dose was used and greater inert gas Flow.

Spraying the mixture at the initial granulation stage [from 0 to 5 minutes]:

- Inert gas Flow: 20-25 [m³/h];

- Slit width: 8-12mm; Atomizing nozzle diameter: 1.2mm;

- Temperature of inert inlet gas: 25.0-29.0 [°C] - in the most preferred variant of the invention, the inert gas temperature should be 25°C, however, the use of higher temperatures, even up to 29°C, does not negatively affect the course of this stage;

- Mixture dose: 15 [g/min];

- Atomizing pressure [1.0 bar] and atomizing nozzle with a diameter of 1.2 mm;

- Maximum bed temperature: 36.0 [°C] Although in the most preferred variant of the invention, the bed temperature should not exceed 34°C;

- Filter shaking pause 20 [s],

- Filter shaking time 10 [s].

The preliminary granulation stage is of key importance when working with input material that is characterized by a very high percentage of particles smaller than 0.125 mm (and such materials, due to their too-small particle sizes, do not qualify For effective, high-efficiency, and uniform protection with a cover). Pre-granulation allows For increasing the particles of the input material to the appropriate size, homogenizing it in terms of particle size, giving it a particle shape close to a sphere, which translates into a more effective application of a non-hydrophilic protective cover in terms of tightness. Therefore, the use of preliminary granulation makes it possible to prepare virtually any material containing probiotic bacteria For the actual coating stage. Another advantage is the Fact that thanks to the use of a cooling stage after initial granulation, it is possible to use only one dual-function, non-hydrophilic cover to achieve two different effects, i.e. granulation (increasing the size, adapting the shape of the particle, partial protection) and coating (giving the target protective and security properties). With this solution, there is no need to separately develop a granulating and coating agent with different compositions, it shortens the total process time of obtaining a protected particle, which translates into an increase in the proFitability of the process and makes the process noninvasive, without affecting the quality of the bacterial material. During the next minutes of the initial granulation stage, to maintain the most correct circulation of the bed and to accelerate the granulation effect, change the parameters according to the guidelines below and continue until the amount of granulating mixture corresponding to 50% (w/w) of the mass of the lyophilizate batch is sprayed.

Spraying the granulating mixture at the initial granulation stage [from 5 to 12-23 minutes depending on the size of the bed at the start and the dose]:

- Inert gas Flow: 25-45 [m³/h] - in the most preferred variant of the invention, it is necessary to gradually increase the volume of the inlet inert gas to maintain proper bed circulation;

- Slit width: 12-20mm;

- Atomizing nozzle diameter: 1.2mm;

- Temperature of inert inlet gas: 25.0 [°C] - in the most preferred variant of the invention, the inert gas temperature should be 25°C, however, the use of higher temperatures, even up to 29°C, does not negatively affect the course of this stage;

- Dose of the hydrophobic mixture: 20 [g/min] - in a less Favorable variant of the invention, the dose may remain at 15g/min;

- Atomizing pressure 1.0 [bar] + atomizing nozzle with a diameter of 1.2mm;

- Maximum bed temperature: 36.0 [°C] Although in the most preferred variant of the invention, the bed temperature should not exceed 34°C;

- Filter shaking pause 20 [s], Filter shaking time 10 [s]

In a specific embodiment of the invention, applying 50% (w/w) of the complex hydrophobic mixture in relation to the amount of the input material is sufficient to give it the Form of Fine particles, which, due to their more spherical shape, undergo the granulation process more effectively with the protective hydrophobic mixture at a later stage. After applying this amount, the particles should be pre-cooled.

### Stage 4 - Pre-cooling of the formed particles

Pre-cooling of the Formed particles covers the time From the moment the spray is stopped, in a preferred embodiment of the invention after 12-14 minutes of spraying, in a less preferred example after 16-17 minutes of spraying, until the bed reaches a temperature below 30°C. In the most preferred variant, the pre-cooling stage lasts 10 minutes and the bed is kept below 30°C For 2 minutes. Parameters For the cooling stage:
Pre-cooling of the particles [from 12-23 minutes to 22-33 minutes depending on the size of the bed at the start and the dose]:

- Inert gas Flow: 35-45 [m³/h];

- Slit width: 12-20mm; Temp. inert inlet gas: 25.0 [°C];

- Dosage of the protective mixture: 0 [g/min];

- Atomizing pressure: None;

- Bed temperature: below 30.0 [°C];

- Filter shaking pause 20 [s], Filter shaking time 10 [s]

### Stage 5 - Proper process with a protective hydrophobic layer

For the proper application of the bifunctional granulating and protective layer on the pre-hardened particles to proceed without any problems, it is necessary to ensure that the pipe supplying the mixture and the spray gun are clear. This effect can be achieved by interrupting the spray by pulling out the end of the supply pipe, which is immersed in the bottle with the melted mixture. The pressure inside the Wurster coating system should suck in all the mixture remaining in the tube and atomizing nozzle within one minute without clogging it. In the most advantageous variant of this stage, the atomizing air supply should be disconnected during the initial cooling (immediately after unblocking the spraying system), which will shorten the initial hardening stage.

After the initial hardening of the surface of the particles, the remaining amount of the hydrophobic protective mixture may be applied. Using the parameters below, spray the amount of hydrophobic system corresponding to the remaining 50% (w/w) of the mass of the lyophilized lactic acid bacteria batch.

The proper process with the protective mixture [from 22-33 minutes to 38-56 minutes depending on the size of the bed at the start and the dose at earlier stages]:

- Inert gas Flow: 35-45 [m³/h] - in the most preferred variant of the invention, it is necessary to gradually increase the volume of the inlet inert gas to maintain proper bed circulation;

- Slit width: 20-22mm;

- Atomizing nozzle diameter: 1.2mm;

- Temperature of inert inlet gas: 25.0 [°C] - in the most preferred variant of the invention, the temperature of the inert gas should be 25 degrees C, however, the use of higher temperatures, even up to 29 degrees C, does not negatively affect the course of this stage;

- Dose of hydrophobic mixture: 15-20 [g/min];

- Atomizing pressure [1.0bar] + atomizing nozzle with a diameter of 1.2mm;

- Maximum bed temperature: max. 34.0 [°C] although in the most preferred variant of the invention, the bed temperature should not exceed 33.5°C;

- Filter shaking pause 20 [s], Filter shaking time 10 [s]

The use of a low atomizing pressure of 1.0 bar at all stages preceding the actual process is intended to eliminate the mechanical stress on bacteria resulting From the point action of compressed air on the surface of lyophilized cells currently located at the outlet of the atomizing nozzle. When higher pressures are used, lyophilized bacterial cells could be subject to mechanical damage, which in turn would reduce their biological activity. In turn, the use of low atomizing pressure at the stage of actual protection is intended to ensure that the protective mixture is sprayed in the Form of the largest possible drop, which results in more accurate coverage of the input material and a more uniform surface of the protected material after spraying the target amount of the protective layer. Moreover, obtaining a larger drop of sprayed cover ensures that the material does not solidify too early before it even reaches the surface of the material particles subjected to preliminary process and proper protection.

In this embodiment of the invention, an amount of a complex, dual-function protective, and granulating cover is sprayed onto the lyophilizate of lactic acid bacteria, which corresponds to 100% (w/w) in relation to the weight of the batch.

Due to the optimal selection of parameters For the actual process stage, mainly the dose and bed temperature, it is possible to use the same hydrophobic mixture both For the initial process of the input material (uncoated lyophilizate) and For coating these particles. Maintaining the bed temperature during the actual coating stage below the melting temperature specified For the component of the mixture with the lowest melting point prevents the previously Formed particles From melting, giving the effect of encapsulation and not Further granulation.

The Final cooling step should be carried out analogously to the pre-cooling step described above.

Final cooling of the protected particles [from 38-56 minutes to 48-66 minutes depending on the size of the bed at the start and the dose at earlier stages]:

- Inert gas Flow: 35-45 [$m^3/h$];

- Slit width: 12-20mm;

- Temperature of inert inlet gas: 25.0 [°C];

- Dose of hydrophobic mixture: 0 [g/min];

- Atomizing pressure: None;

- Bed temperature: below 30.0 [°C];

- Filter shaking pause 20 [s], Filter shaking time 10 [s].

**Final steps and storage**

The Final semi-finished product in the Form of protected particles is sieved through a sieve with a mesh size of 1.0 mm to remove particles that are too large. Losses after sieving of the microcapsule Fraction larger than 1.0 mm are below 0.1% (w/w) in relation to the amount of the Final semi-finished product. To avoid reducing the biological activity in the produced material before applying them to a Food product (fortified Food, Food For special medical purposes, Food For people with specific nutritional needs in the prevention of lifestyle diseases, including overweight and obesity, as well as in case of non-specific inflammatory bowel diseases or athletes' diets) they are placed in a double polyethylene bag From where the air is removed. The pre-protected semi-finished product is additionally placed in a bag with an aluminum layer and a ZIP closure and then placed in the refrigerator (2-8°C). In a more preferred variant, the semi-finished product packed in this way is placed in a Freezer (-20°C). Thanks to this procedure, the durability of the semi-finished product in the Form of protected particles is extended to the maximum possible extent after application to the Food product, as losses of biological activity are prevented before being used For Food production.

**Process efficiency**

The process of protecting lactic acid bacteria with a complex hydrophobic layer, which is the subject of the invention, is characterized by high efficiency, not less than 90%. In the most preferred embodiment, not less than 93%. This means that as a result of a single process of protecting lactic acid bacteria with a complex hydrophobic layer, From 1200 to 1300 g of microcapsules with increased resistance to moisture and oxidizing Factors are obtained, using a Wurster column with a volume of 6 L For the process. During an 8-hour working day, it is possible to produce From 2,400 to 2,600 g of single-layer microcapsules. Increasing the scale by using attachments with a larger volume will proportionally increase the number of single-layer microcapsules produced per unit of time. The duration of the processing of the input material, taking into account only the stages of exposure of the lyophilizate to stress Factors, is no more than 70 minutes.

**The impact of the process on the input material**

The non-invasive nature of the discussed process of protecting lactic acid bacteria with a complex mixture was proven thanks to the comparative analysis of the results obtained For samples of the input material (lyophilizate) and the Final semi-finished product (single-layer microcapsules) in terms of parameters such as:

a) viability, which was determined using Flow cytometry, where the result presented as a percentage expresses the ratio of living cells to the total number of cells in the tested material. The total number of cells in the probiotic material also includes dead and damaged cells

b) water activity

c) Free and bound water content - determined by the Karl-Fischer method, expressed in [%]

d) ability to Form colonies under Favorable incubation conditions using the plate culture method, where the result is presented as the number of units capable of Forming colonies From one gram of material [CFU/g]. In the case of the produced protected particles, the expected number of units capable of Forming colonies From 1 g was determined by taking into account the percentage of the applied protective layer in the result For the input material (lyophilizate) For a given series.

## Example 2

The comparative analysis of the above-mentioned parameters performed For the input material For each separate series as well as For the microcapsules produced in these series showed that:

(i) The lactic acid bacteria protection process performed according to a specific embodiment of the invention does not lead to a decrease in viability in the Final semi-finished product compared to the viability expressed as a percentage of the input material. The viability values recorded For the lyophilized product ranged From 64 to 70%, depending on the batch of raw material used. In turn, the lifetimes recorded For protected particles where the analysis was performed immediately after the process were not lower than the percentage values recorded For the input material.

*Service life recorded for protected particles*

**Series No. 1**: lyophilisate life = 69.82% -> durability of the protected particles = 78.65%

**Series No. 2:** lyophilisate lif. = 64.59% -> durability of protected particles = 73.91%

**Series No. 3:** lyophilisate life = 66.64% -> durability of the protected particles = 74.32%

**Series No. 4:** lyophilisate lif. = 66.64% -> durability of the protected particles = 76.53%

(ii) The developed protocol For handling the input material and the protection process itself do not lead to an increase in the water content in the semi-finished product, which could prematurely start the process of waking up lactic acid bacteria cells From a lyophilized state, and the amount of water that can be determined by the Karl-Fischer method in the protected particles is much lower than the amount of water that can be determined in the input material. This proves that the applied protective layer fulfills its Function, which significantly limits the possibility of moisture penetration not only into the microcapsule but also From its interior during the Karl-Fischer test, as this direction of water migration is used in this method.

*Water content in the protected particles*

**Series No. 1:** lyophilisate water content. = 1.290% -> water content in protected particles = 0.480%

**Series No. 2:** lyophilisate water content. = 1.526% -> water content in protected particles = 0.548%

**Series No. 3:** lyophilisate water content. = 1.334% -> water content in protected particles = 0.656%

**Series No. 4:** lyophilisate water content. = 1.254% -> water content in protected particles = 0.522%

(iii) The process does not result in an increase in water activity in the semi-finished product. This proves that the correct protocol For handling the input material has been developed before the protection stage and that the atmosphere For this process is appropriately selected. The lack of increase in water activity combined with the increased resistance to ambient moisture resulting From the application of the protective layer contributes to the extension of the durability of the material by delaying the emergence of lactic acid bacteria cells From the state of anabiosis during storage in conditions of increased air humidity.

*Water activity content (aw) of the protected particles after the process*

**Series No. 1:** aw lyoph. = 0.0647% -> content of protected particles after the process - 0.0627%

**Series No. 2:** aw lyoph. = 0.0627% -> content of protected particles after the process - 0.0570%

**Series No. 3:** aw lyoph. = 0.1281% -> content of protected particles after the process - 0.1088%

**Series No. 4:** aw lyoph. = 0.1281% -> content of protected particles after the process - 0.0899%

(iv) The process of securing the lyophilized product does not impair lactic acid bacteria cells in terms of their ability to Form colonies, as shown by the results obtained For the protected particles immediately after the process. The expected number of units capable of Forming colonies From 1 g of the Final semi-finished product was determined by taking into account the percentage of the applied protective layer in the result For the input material to a given series, which in a specific embodiment of the invention is 50%.

*Number of units capable of creating colonies*

**Series No. 1:** amount of CFU/g in the input material = $4.90 \times 10^{10}$ -> expected amount of CFU/g in particles = $2.45 \times 10^{10}$ -> result obtained For protected particles = $3.4 \times 1010$

**Series No. 2:** amount of CFU/g in the input material = $2.30 \times 10^{11}$ -> expected amount of CFU/g in particles = $1.15 \times 10^{11}$ -> result obtained For protected particles = $1.3 \times 10^{11}$

**Series No. 3:** amount of CFU/g in the input material = $7.33 \times 10^{11}$ -> expected amount of CFU/g in particles = $3.67 \times 10^{11}$ -> result obtained For protected particles = $3.5 \times 10^{11}$

**Series No. 4:** amount of CFU/g in the input material = $6.00 \times 1011$ -> expected amount of CFU/g in particles = $3.00 \times 10^{11}$ -> result obtained For protected particles = $3.0 \times 10^{11}$

*Example 3*

The thermal resistance of the obtained particles, protected with a specific bifunctional cover, was proven by the results of stability tests immediately after enriching it with dark chocolate ingredients (where the enrichment consisted of mixing the protected particles in dark chocolate heated to a temperature of 32-34°C For about 30 minutes and then Forming From it, thanks to dosing systems equipped with nozzles of a specific diameter, Fine sprinkles in the shape of irregular dragees) as well as during 12-month stability tests. The test results showed lower losses of bacterial material as a result of enrichment with dark chocolate ingredients and extended durability in stability tests in the case of particles protected with a dual-function cover with the addition of cocoa Fat. The reported results are presented in Fig. 2.

**Example 4**

The results verifying the non-invasive nature of the protection process and extended durability were obtained thanks to the horizontal method of determining the number of lactic acid bacteria using the plate method described below:

Equipment used: incubator capable of maintaining a temperature of 37°C ($\pm$1°C); Petri dishes, made of glass or plastic, 90 mm in diameter; graduated pipettes with nominal capacity of 10 ml and 1 ml, calibrated with graduations of 0.5 ml and 0.1 ml; water bath or similar apparatus capable of maintaining a temperature of 47°C ($\pm$2°C); pH meter with a sensitivity of 0.01 pH unit at 25°C ($\pm$0.1 pH unit); scale For preparing samples with an accuracy of at least 0.01 g;

Reagents used: buffered peptone water; MRS agar (de Man, Rogosa, and Sharpe);

Composition of buffered peptone water: peptone; sodium chloride; sodium hydrogen phosphate; potassium hydrogen phosphate; water;

composition of MRS agar (de Man, Rogosa, and Sharpe): enzymatic casein hydrolyzate; meat extract; yeast extract; triammonium citrate; sodium acetate; magnesium sulfate 7-hydrate; manganese sulfate-4-hydrate; dipotassium hydrogen phosphate; glucose; sorbitan monooleate; agar; water.

**Preparation of the starting suspension**

Prepare the initial suspension in accordance with the PN-EN ISO 6887-1 standard by weighing 10 g of the sample into 90 ml of buffered peptone water (pH 7 $\pm$ 0.2 at 25°C) to obtain a 1:10 dilution. Homogenize the prepared sample using a paddle homogenizer to obtain a homogeneous suspension. Make Further ten-fold dilutions in buffered peptone water, adjusting the number of serial dilutions to the expected number of lactic acid bacteria.

**Inoculation and incubation**

Inoculation and incubation should be carried out in accordance with the PN-ISO 15214 standard at the optimal temperature For the growth of *Lactobacillus* spp bacteria (PN-EN 15787). Using a sterile pipette, transfer 1 ml of the appropriate dilution of the tested sample to two sterile Petri dishes. Pour 15 ml of previously dissolved and cooled MRS agar medium at 47°C in a water bath into each Petri dish. Mix the liquefied medium with the seeds and allow to solidify. The Final pH of the medium should be 5.7 ($\pm$0.1) at 25°C. Incubate the completely solidified medium with the tested material in anaerobic conditions by placing the plates in airtight bags or containers with generators and anaerobic zone indicators. Invert the inoculated plates and incubate in an incubator at 37°C ($\pm$1°C) For 72 hours.

**Calculating the number of bacteria**

For bacterial count calculations, select plates containing less than 300 colonies From two consecutive dilutions and more than 15 colonies on at least one plate.

Calculate the N number of lactic acid bacteria in the test sample as a weighted average taking into account two successive dilutions according to the Following equation:

$$N = \frac{\sum C}{V(n_1 + 0{,}1 n_2)d}$$

where: $\sum C$ - sum of colonies on all plates From two consecutive dilutions; V - volume of inoculation applied to each plate; n - number of plates From the First counted dilution; d - dilution Factor corresponding to the First dilution counted; results should be rounded to two significant digits in accordance with ISO 7218.

**Storage test results**

The increased resistance of the developed composition to elevated temperature and humidity conditions compared to the commercial, microencapsulated Form of lyophilizate containing lactic acid bacteria was proven in long-term storage tests lasting 12 months.

Storage tests were carried out in climatic chambers with a temperature of 25°C and a relative air humidity of 60%. These conditions corresponded to climatic zone I according to the standardization defined by WHO and significantly exceeded in their absolute values the conditions recommended For materials containing lactic acid bacteria when producing Food (enriched, Food For special medical purposes, Food For people with specific nutritional needs in the prevention of lifestyle diseases), including overweight and obesity, as well as in inflammatory bowel diseases or athletes' diets) and then storing them to extend the shelf life of the Finished product.

Samples of the developed composition of protected particles and commercial microencapsulated lyophilizate of *L. rhamnosus* were packed according to specific rules to ensure identical storage conditions:

(i) individual samples of both tested materials contained the same amount of material

(ii) the same packaging materials were used For both materials, i.e. a polyethylene bag with aspirated air + Foil with an oxygen barrier (OTR $\leq$ 0.1 [cm$^3$/(m$^2 \times$ 24h)] and a moisture barrier (WVTR $\leq$ 0 .1 [g/(m$^2 \times$24h)]

(iii) samples of both materials were removed From the climatic chambers after the same storage periods (0, 3, 6, 9 and 12 months).

The results of long-term stability tests (25 degrees C/60% humidity) conducted For the materials specified above, where the result was expressed in the colony-forming capacity unit [CFU/g], are as Follows:

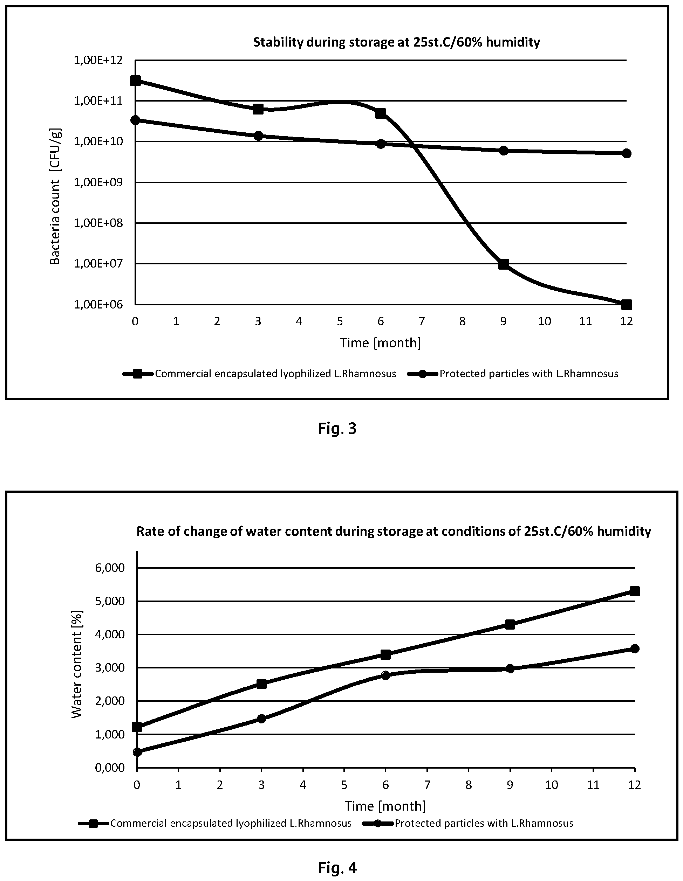

(i) In the case of a composition of protected particles surrounded by a non-hydrophilic protective layer where the result was expressed per 1 g of material containing 0.5 g of lactic acid bacteria, the results are presented in Figure 3: point "0" at the start of the tests: $3.4 \times 10^{10}$ CFU/g; after 3 months: $1.4 \times 10^{10}$ CFU/g; after 6 months: $8.9 \times 10^9$ CFU/g; after 9 months: $6.1 \times 10^9$ CFU/g; after 12 months: $5.2 \times 10^9$ CFU/g.

(ii) In the case of a composition of protected particles surrounded by a non-hydrophilic layer of a protective layer, where the result was expressed as the amount of material containing 1g of lactic acid bacteria, the results are presented in Figure 3: point "0" at the start of the tests: $6.8 \times 10^{10}$ CFU/2g; after 3 months: $2.8 \times 10^{10}$ CFU/2g; after 6 months: $1.78 \times 10^{10}$ CFU/2g; after 9 months: $1.22 \times 10^{10}$ CFU/2g; after 12 months: $1.04 \times 10^{10}$ CFU/2g.

(iii) In the case of a commercial microencapsulated Form of *L. rhamnosus* lyophilizate, where the result was expressed per 1g of lyophilizate containing 1g of lactic acid bacteria, the results are presented in Figure 3: point "0" at the start of the tests: $3.2 \times 10^{11}$ CFU/g; after 3 months: $6.4 \times 10^{10}$ CFU/g; after 6 months: $5.0 \times 10^{10}$ CFU/g; after 9 months: $1.0 \times 10^7$ CFU/g; after 12 months: $<1.0 \times 10^7$ CFU/g

The analysis of the presented results indicates the achievement of increased durability during storage in conditions of increased humidity and temperature in the particles protected by a complex, dual-functional granulating, and protective cover, which is the subject of the invention.

**Example 5**

The extended durability of the protected particles is also the result of a lower rate of increase in water content (determined by the Karl-Fischer method) and water activity during its storage compared to the rate of increase in these parameters in a commercial, microencapsulated lyophilizate of lactic acid bacteria. However, the reduction in growth rate in the case of water activity was lower than in the case of water content. Applying a complex protective layer to the input material gave the Final semi-finished product a less hygroscopic character (hygroscopicity of the unencapsulated lyophilizate - 22%, the hygroscopicity of the protected particles - 9%), which resulted in the slowing down of the processes of moisture penetration into the interior of the particles, thus slowing down the processes of waking up lyophilized cells and, consequently, reducing their biological activity and dying. As confirmation, changes in water content in samples of protected particles and commercial microencapsulated Freeze-dried products throughout long-term storage tests were presented.

The rate of changes in water content in particles, commercial lyophilizate of *L. rhamnosus,* the results of which are presented in Fig. 4:

- point "0" at the start of the research: 1.231%

- after 3 months: 2.523% (iii)

- after 6 months: 3.408% (iv)

- After 9 months: 4.312% (v)

- after 12 months: 5.310%

The rate of changes in water content in particles containing *L. rhamnosus* lactic acid bacteria protected with a complex non-hydrophilic layer, the results of which are presented in Fig. 4:

- point "0" at the start of the research: 0.480%

- after 3 months: 1.475%

- after 6 months: 2.778%

- after 9 months: 2.978%

- after 12 months: 3.570%

**Example 6**

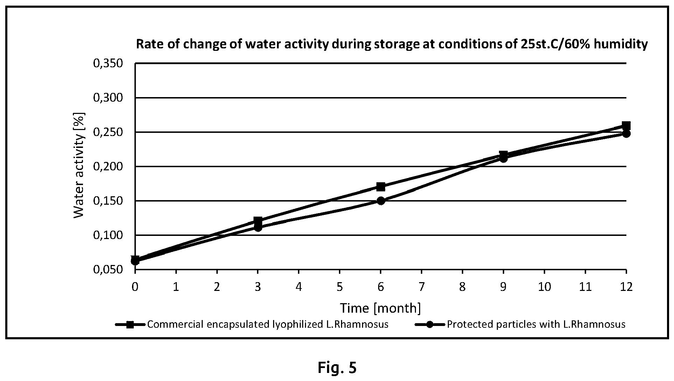

The rate of changes in water activity in particles, commercial lyophilizate of *L. rhamnosus,* the results of which are presented in Fig. 5:

- point "0" at the start of the research: 0.0647%

- after 3 months: 0.1211%

- after 6 months: 0.1711%

- after 9 months: 0.2166%

- after 12 months: 0.2595%

The rate of water activity of the above-mentioned particles containing *L. rhamnosus* lactic acid bacteria protected with a complex non-hydrophilic layer, the results of which are presented in Figure 5:

- point "0" at the start of the research: 0.0627%

- after 3 months: 0.1116%

- after 6 months: 0.1505%

- after 9 months: 0.2120%

- after 12 months: 0.2484%

A semi-finished product in the Form of protected particles containing probiotic bacteria is used in applications to product Forms such as chocolate, chocolate products, bars, semi-liquid and liquid products, capsules, powders, and particles. Thanks to its high thermal resistance, these particles can be used in chocolate products.

**Claims**

1. Particles containing Freeze-dried lactic acid bacteria, covered with a single, three-component hydrophobic layer of plant origin, **characterized in that**, the hydrophobic layer consists of a mixture oF:

    a. cocoa butter NCB-HDO3-473,
    b. two saturated Fatty acids,

    and the cocoa Fat content in the hydrophobic layer is in the range of 25-29% by weight.

2. Particles according to claim 1, **characterized in that**, the lactic acid bacteria belong to the *Lactobacillus rhamnosus* GG strain (ATC53103) and the total amount of Freeze-dried bacteria in the particles is 45-55% by weight, and the hydrophobic layer is 45-55% by weight particles.

3. Particles according to any of claim 1 or 2, **characterized in that**, the melting point of the hydrophobic layer is in the range From 34°C to 64°C.

4. Particles according to any of claims 1-3, **characterized in that**, one of the saturated Fatty acids has a melting point in the range of 57-64 °C, the other saturated Fatty acid has a melting point in the range of 53-60 °C, and the cocoa Fat NCB-HDO3-473 has a melting point in the range of 34-36 °C.

5. Particles according to any of claims 1-4, **characterized in that**, the content of cocoa Fat, Fatty acid with a lower melting point, and Fatty acid with a higher melting point was selected according to the weight proportion, respectively: 1.125: 1.0: 2, 0416.

6. Particles according to any one of claims 1-5, **characterized in that**, the saturated Fatty acids are selected From the group consisting of palmitic acid and stearic acid.

7. A method For producing particles containing lactic acid bacteria, with the size of a single particle in the range of 0.125 mm -1.0 mm including the Following steps:

    a) preparation of a hydrophobic coating mixture consisting of cocoa butter and two saturated Fatty acids,
    b) conditioning of lactic acid bacteria by heating to a temperature of 20-22 ° C For at least 2 hours,
    c) carrying out preliminary granulation,
    d) cooling,
    e) carrying out proper granulation,

the total yield of the process is at least 90% and the water content in the obtained particles is does not exeed 0.7%.

**8.** The method according to claim 7, **characterized in that**, step a) includes melting all the ingredients, wherein melting is carried out in the order resulting From the melting temperatures of individual ingredients From the lowest to the highest, at a temperature of 110°C, and then sterilization of the molten mixture is carried out at 121°C.

**9.** The method according to claim 7 or 8, **characterized in that** the content of individual coating components is in the weight proportion: 1.125: 1: 2.042, respectively: cocoa butter, Fatty acid with a lower melting point, and Fatty acid with a higher melting point.

**10.** The method according to any of claims 7 - 9, **characterized in that** the unsaturated Fatty acids are selected From the group consisting of palmitic acid and stearic acid.

**11.** A method according to any of claims 7 - 10, **characterized in that** step c) is carried out by hot melting in a Wurster coating system providing spray From below or in a Fluidized chamber providing spray From above
and consists of two stages:

    i) the duration of the First stage is 5 minutes, the spraying dose of the mixture From point a) is 15 g/min, the inert gas Flow is 20-25 $m^3$/h, the atomizing pressure is 1 bar, the inlet inert gas humidity is in the range of 0, 0-0.1g/kg,
    ii) the duration of the second stage is From 7 to 18 minutes, the spraying dose of the mixture From point a) is 20 g/min. inert gas Flow: 25-45 m3/h, atomizing pressure is 1 bar, inlet inert gas humidity is in the range of 0.0-0.1g/kg

**12.** Method according to any of claims 7 - 11, **characterized in that** cooling in step d) is carried out in an inert gas Flow of 35-45 $m^3$/h For 10 minutes, the humidity of the inlet inert gas is in the range of 0.0-0, 1g/kg.

**13.** A method according to any of claims 7 - 12, **characterized in that** granulation in step e) takes place within 16 to 23 minutes, the spray dose of the mixture From point a) is 15-20 g/min, the inert gas Flow is 35-45 $m^3$/h, atomizing pressure is 1 bar, inlet inert gas humidity is in the range of 0.0-0.1g/kg.

**14.** A method according to any of claims 7-13, **characterized in that** the percentage distribution of Fractions with different particle sizes is as Follows: the Fraction with the size of a single protected particle in the range of 0.16 mm - 0.6 mm - constitutes 85% by weight of the total material, the Fraction with a particle size in the range of 0.125 mm-0.16 mm - constitutes less than 4% of the total, and the Fraction with a particle size in the range of 0.6 mm - 0.8 mm - constitutes 10-11% by weight of the total material.

**15.** Use of particles obtained by the method according to claims 7-14, in product Forms such as chocolate, chocolate products, bars, products in liquid and semi-liquid Form, capsules, and sachets with ZIP or stick closures.

Fig. 1

Fig. 2

Fig. 3

Fig. 4

**Fig. 5**

# EP 4 548 770 A1

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/172271 A1 (AMD PHARMA LTD [IL]) 18 August 2022 (2022-08-18) <br> * claim 1 * <br> * claim 7 * <br> * claim 9 * <br> * claim 11 * <br> * claim 14 * <br> * claim 15 * <br> * page 13, line 13 – line 14 * <br> * page 18, line 17 – line 30 * <br> * page 34, line 11 – line 19 * <br> * page 40, line 6 * | 1-15 | INV. <br> A23G1/42 <br> A23L33/135 <br> A23P10/35 <br> A23P20/10 <br> A61K9/50 <br> A61K35/744 <br> A61K35/747 <br> C12N1/04 <br> C12N11/02 |
| X | US 2021/186887 A1 (PENHASI ADEL [IL] ET AL) 24 June 2021 (2021-06-24) <br> * claim 1 * <br> * claims 4-6 * <br> * paragraph [0013] * | 1-6 | |
| A | US 2013/323362 A1 (PENHASI ADEL [IL]) 5 December 2013 (2013-12-05) <br> * claim 1 * | 1-6 | TECHNICAL FIELDS SEARCHED (IPC) |
| A | US 2003/109025 A1 (DURAND HENRI [FR] ET AL) 12 June 2003 (2003-06-12) <br> * paragraph [0046] * | 1-6 | A23G <br> A61K <br> C12R <br> A23P <br> A23L <br> C12N |

The present search report has been drawn up for all claims

| Place of search | Date of completion of the search | Examiner |
|---|---|---|
| The Hague | 13 March 2024 | Alonso Martínez, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 7769

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2024

| Patent document cited in search report | Publication date | Patent family member(s) | Publication date |
|---|---|---|---|
| WO 2022172271 A1 | 18-08-2022 | EP 4291210 A1 | 20-12-2023 |
| | | WO 2022172271 A1 | 18-08-2022 |
| US 2021186887 A1 | 24-06-2021 | AU 2019255435 A1 | 26-11-2020 |
| | | BR 112020021458 A2 | 19-01-2021 |
| | | CA 3097489 A1 | 24-10-2019 |
| | | CN 112292118 A | 29-01-2021 |
| | | EA 202092347 A1 | 17-02-2021 |
| | | EP 3781137 A1 | 24-02-2021 |
| | | IL 278101 A | 30-11-2020 |
| | | JP 2021521249 A | 26-08-2021 |
| | | KR 20210003138 A | 11-01-2021 |
| | | US 2021186887 A1 | 24-06-2021 |
| | | WO 2019202604 A1 | 24-10-2019 |
| US 2013323362 A1 | 05-12-2013 | AU 2011340174 A1 | 25-07-2013 |
| | | BR 112013014044 A2 | 31-10-2017 |
| | | CA 2820178 A1 | 14-06-2012 |
| | | CN 103347395 A | 09-10-2013 |
| | | EP 2648528 A1 | 16-10-2013 |
| | | ES 2597038 T3 | 13-01-2017 |
| | | PT 2648528 T | 25-10-2016 |
| | | US 2013323362 A1 | 05-12-2013 |
| | | US 2016360777 A1 | 15-12-2016 |
| | | WO 2012077038 A1 | 14-06-2012 |
| US 2003109025 A1 | 12-06-2003 | AT E394472 T1 | 15-05-2008 |
| | | AU 4426101 A | 24-09-2001 |
| | | DK 1265983 T3 | 08-09-2008 |
| | | EP 1265983 A1 | 18-12-2002 |
| | | ES 2306707 T3 | 16-11-2008 |
| | | FR 2806417 A1 | 21-09-2001 |
| | | US 2003109025 A1 | 12-06-2003 |
| | | WO 0168808 A1 | 20-09-2001 |

EPO FORM P0459

For more details about this annex : see Official Journal of the European Patent Office, No. 12/82

## REFERENCES CITED IN THE DESCRIPTION

**Patent documents cited in the description**

- EP 3205216 B1
- US 110396372 B2
- WO 2002058735 A1
- US 6900173 B2
- US 4888171 A
- EP 1265983 B1